# EUROPEAN PATENT APPLICATION

(11) **EP 0 723 156 A2**
(43) Date of publication of application: **24.07.1996**
(21) Application number: 95119239.2
(22) Date of filing: 06.12.1995
(51) Int. Cl.: G01N 33/58, G01N 33/533, G01N 33/74, G01N 33/82

(54) **Assay normalization method**

(30) Priority: 20.12.1994 US 359382
(71) Applicant: BEHRING DIAGNOSTICS INC., Westwood, MA 02090-2399 (US)
(72) Inventor: Bahar, Izak, Sharon, MA 02067 (US); Chiuli, Anthony, Newton, MA 02158 (US); Inbar, Shai, Brookline, MA 02146 (US)
(74) Representative: Reitzner, Bruno, Dr.

(57) **Abstract**

There is provided a method of normalizing the signal of an assay for an analyte in a biological sample comprising (a) adding an internal indicator to at least one of the assay reagents; (b) performing the assay by adding and/or mixing the assay reagents and reading the signal generated by the internal indicator; (c) reading the signal produced by the assay reagents; and (d) calculating the normalized assay signal.

## Description

The present invention relates to a method of normalizing the signal of an analytical assay. More particularly, the present invention relates to a method of normalizing the assay signal of an analytical assay comprising the incorporating of an internal indicator in the reagent system thereby improving the precision of the determination.

Many methods for the determination of analytes in biological fluids are known in the art. These generally involve the interaction of an analyte with various reagents, resulting in a detectable change in the characteristics of the reagent system. For example, interaction between the analyte and the reagents may result in a color change in the system that can be detected and evaluated visually or read spectrophotometrically. In addition, spectrofluorometry may be employed to detect a fluorescent signal generated by the interaction of a labelled biological species. All of the methods involve the addition of biological samples to reagents, either manually, or automatically in a diagnostic instrument. In each, a biological fluid is added to the reagent systems generally in liquid form or in solutions, by pipetting. To determine the analyte, i.e., to quantify the analyte in the biological fluid, known, specific volumes and/or concentrations of the liquid sample and reagents must be employed. While modem pipettes for manual additions, and pipetting devices for automated additions, generally provide precise amounts of the sample and reagents, even when small volumes are involved, significant imprecision may occur. Imprecisions may result from misuse of the pipettes by the operator or malfunctions of the device. In manual pipetting, for example, imprecision may result from the use of faulty or miscalibrated pipettes. In automatic pipetting, imprecison may result from, not only the use of faulty equipment, but from the failure of the pipette to withdraw the correct amount of the liquid sample or reagent for subsequent adding and mixing. Air bubbles in the liquid sample or reagents will cause pipetting imprecision in the assay.

We have now found that by incorporating an internal indicator in the reagent system, the effects of the variability of the volumes and/or concentrations of the liquid sample and/or reagents on the precision of the determination of the analyte in the biological sample is minimized, thus leading to a more accurate evaluation of an analyte of interest. To be effective as an internal indicator in a diagnostic assay, a substance must not interact with the components of the reagent system and sample and must be subject to the same addition (by pipetting) and mixing steps as the active reagents and sample. Under these circumstances, variations in the indicator signal resulting from pipetting imprecisions reflect variations in the analyte concentration and permits correction of the assay signal by normalization thereof.

Among fluorescent internal indicators, there may be mentioned coumarins such as umbelliferone and xanthenes such as N-[9-(2-carboxyphenyl)-6-diethylamino)-3H-xanthen-3-ylidene]-N-ethylethanaminium chloride (Rhodamine B), bis-(3-sulfopropyl)rhodamine carboxylic acid or bis-(3-sulfopropyl)rhodamine piperazinamide.

The present invention, while adaptable to a variety of assays, is suitable for analytical tests for measuring the concentration of analytes in biological samples by immunogenic techniques, involving antibodies specific to the analyte to be determined. These assays, including, for example, competitive assays and enzyme immunoassays involve steps of pipetting and mixing liquid sample and reagents, the variations of the pipetting and mixing steps contributing to the imprecision of the final assay signal. Of interest is the competitive diagnostic assay. In this assay for low molecular weight analytes, an analyte and labeled analyte analog compete for the binding sites of an antibody, or a labeled antibody and immobilized analyte analog compete with an analyte for binding sites. In both, the final analyte signal, and thus the determination of the analyte, is sensitive to variations of the liquid volumes and concentrations of the sample and reagents. Variations in these parameters are magnified in the final signal value, and, thus, the use of an internal indicator is especially beneficial in these determinations..

In practice, as illustrated for a fluoroimmunoassay for estradiol, conducted in an automated instrument such as the OPUS® instrument, manufactured and marketed by Behring Diagnostics, Inc., Westwood, MA (see C. Olive, Journal of Clinical Immunoassay, 14, 126 (1991) for a description of the instrument, the test module and discussion of the mode of operation thereof), the assay is performed in a test module having three foil-sealed wells, separately containing rabbit polyclonal antibody to estradiol, estradiol-alkaline phosphatase conjugate, and 4-methylumbelliferyl phosphate fluorogenic substrate in the wells and incorporating a Whatman glass fiber matrix GF/F thereon, on which goat-anti-rabbit IgG antibody is immobilized. In operation, the pipetting and mixing steps consist of adding fluorescent indicator, bis-(3-sulfopropyl)rhodamine carboxylic acid, to estradiol-alkaline phosphatase conjugate and conjugate-indicator solution and antibody to estradiol. After incubation for forty-five (45) minutes, the assay mixture is dispersed onto the glass fiber matrix and the indicator fluorescence is measured. The assay mixture is washed with 4-methylumbelliferyl phosphate fluorogenic substrate to remove the unbound conjugate. The fluorescence of the bound conjugate-fluorogenic substrate is monitored and the normalized assay signal is calculated by multiplying the ratio of the observed assay signal to the rhodamine signal by a constant.

As shown in Example 1, by the use of the internal indicator, the coefficient of variability (CV) is reduced from 11.55% to 5.16% in the estradiol determination.

Similarly, vitamin B₁₂ in a sample is determined in a fluoroimmunoassay run on the OPUS® automated instrument using a three foil-sealed well module. In this assay, separate wells contain intrinsic factor (a natural protein), supplied by Scrips Laboratories, Inc., San Diego, California, latex particles of 0.8 micron or greater in length, supplied by Polysciences Inc., Warrington, Pennsylvania, and a known concentration of fluorescent indicator, bis-(3-sulfopropyl)rhodamine carboxylic acid, added by pipetting to vitamin B₁₂-alkaline phosphatase conjugate and 4-methylumbelliferyl phosphate fluorogenic substrate. The assay sample, containing vitamin B₁₂ is added to the latex-intrinsic factor solution, and after thorough mixing by subsequent aspirating and pipetting, the solution is incubated for thirty (30) minutes and dispersed over the glass fiber matrix. The signal of the fluorescent rhodamine is then read. Vitamin B₁₂-alkaline phosphatase conjugate is added to the fiber, and then the mixture is washed with 4-methylumbelliferyl phosphate fluorogenic substrate. The bound conjugate turns over the fluorogenic substrate. The fluorescent assay signal is monitored, and the result is normalized as in the estradiol determination. The imprecision of the assay signal is reduced from a coefficient of variability (CV) of 12.3% to 6.7% by this normalization technique.

The reagents used in the present assay are commercially available or are prepared by known methods. For example, bis-(3-sulfopropyl)rhodamine carboxylic acid and bis-(3-sulfopropyl)rhodamine piperazinamide are prepared as described in U. S. Patent 4,900,686, issued February 13, 1990 to M.J. Arnost, et al.

### EXAMPLE 1

### Estradiol assay

30 µl of estradiol alkaline phosphatase conjugate is used in the assay. The conjugate is contained in a sealed well on the test module. Intentionally, 9 out of 20 modules were filled with less volume than the prescribed 30 µl; 25 µl, 20 µl or 15 µl. The same level estradiol 150 pg/ml was run with all the test modules and the signals were measured. The signals measured were either left alone or normalized with the corresponding bis-(3-sulfopropyl)rhodamine carboxylic acid signal value. The variability of the signals was measured by calculating the coefficient of variation (CV): standard deviation/mean for the 20 assay signals.

| Volume | Assay Signal | Rhodamine | *Normalized Signal |
|---|---|---|---|
| | 30.07 | 29.69 | 32.63 |
| | 28.19 | 31.98 | 28.4 |
| | 26.31 | 29.03 | 29.21 |
| 25 µl | 29.39 | 26.93 | 35.17 |
| 20 µl | 23.98 | 23.97 | 32.24 |
| 15 µl | 22.84 | 23.49 | 31.33 |
| | 28.73 | 29.48 | 31.4 |
| | 28.55 | 29.87 | 30.79 |
| | 28.7 | 31.13 | 29.7 |
| 25 µl | 27.01 | 27.59 | 31.54 |
| 20 µl | 24.43 | 23.35 | 33.71 |
| 15 µl | 22.83 | 22.75 | 32.32 |
| | 26.33 | 28.66 | 29.59 |
| | 27.17 | 28.17 | 31.07 |
| | 28.62 | 29.04 | 31.75 |
| 25 µl | 28.42 | 28.83 | 31.75 |
| 20 µl | 21.04 | 23.47 | 28.88 |
| 15 µl | 19.13 | 19.61 | 31.44 |
| | 28.14 | 29.14 | 31.11 |
| | 28.99 | 29.85 | 31.29 |
| Mean | 26.44 | | 31.27 |
| Stdev | 3.06 | | 1.61 |
| %CV | 11.55 | | 5.18 |

| | | | |
|---|---|---|---|
| * Normalized Signal = (Assay Signal/Rhodamine Signal) x Constant | | | |

### EXAMPLE 2

### OPUS B₁₂ assay

50 µl of latex-intrinsic factor complex is used in the assay. The latex is contained in a scaled well on the test module. The same level of vitamin B₁₂ 1150 pg/ml was run with all the test modules and the signals were measured. The signals measured were left alone or normalized with the corresponding bis-(3-supfopropyl)rhodamine carboxylic acid signal value. The variability of the signals was measured by calculating the coefficient of variation (CV); standard deviation/mean for the 18 assay signals.

| | Assay Signal | Rhodamine | *Normalized Signal |
|---|---|---|---|
| | 5.29 | 5.23 | 4.86 |
| | 4.79 | 5.15 | 4.47 |
| | 5.54 | 5.53 | 4.81 |
| | 4.46 | 4.92 | 4.35 |
| | 4.97 | 5.01 | 4.77 |
| | 5.01 | 5.14 | 4.68 |
| | 3.92 | 4.78 | 3.94 |
| | 5.05 | 5.34 | 4.54 |
| | 4.65 | 4.99 | 4.48 |
| | 8.05 | 6.14 | 4.73 |
| | 4.31 | 4.82 | 4.29 |
| | 6.17 | 6.19 | 4.79 |
| | 4.06 | 5.03 | 3.88 |
| | 5.21 | 5.38 | 4.64 |
| | 4.96 | 5.74 | 4.15 |
| | 4.34 | 5.02 | 4.15 |
| | 4.85 | 5.18 | 4.49 |
| | 5.07 | 5.70 | 4.27 |
| Mean | 4.93 | | 4.46 |
| Stdev | 0.61 | | 0.30 |
| %CV | 12.3 | | 6.70 |

| | | | |
|---|---|---|---|
| * Normalized Signal = (Assay Signal/Rhodamine Signal) x Constant | | | |

The imprecision of the assay signal before correction was 12.3% CV and after normalization the CV decreased to 6.7%.

## Claims

1. A method of normalizing the signal of an assay for an analyte in a biological sample comprising:
a. adding an internal indicator to at least one of the assay reagents;
b. performing the assay by adding and/or mixing the assay reagents and reading the signal generated by the internal indicator;
c. reading the signal produced by the assay reagents;
d. calculating the normalized assay signal.

2. The method of claim 1 wherein the precision of the assay is improved.

3. The method of claims 1 or 2 wherein the internal indicator is a fluorescent indicator which is preferably selected from the group consisting of a coumarin and a xanthene or derivative thereof.

4. The method of claim 3 wherein the coumarin is 4-methylumbelliferone.

5. The method of claim 3 wherein the xanthene is N-[9-(2-carboxyphenyl)-6-diethylamino)-3H-xanthen-3-ylidene]-N-ethylethanaminium chloride (Rhodamine B), bis-(3-sulfopropyl) rhodamine carboxylic acid or bis-(3-sulfopropyl)rhodamine piperazinamide.

6. The method of any one of claims 1 to 5 wherein the assay is an immunoassay, preferably an enzyme immunoassay or a competitive assay.

7. The method of claim 6 wherein an analyte, an antibody specific to the analyte, a conjugate, an anti-antibody specific to the antibody, and a fluorescent substrate are employed.

8. The method of claim 7 wherein an internal indicator-conjugate solution analyte mixture is added by pipetting to an antibody, the solution is incubated and the indicator signal is read.

9. A method of normalizing the signal of an immunoassay involving pipetting of liquid reagents and samples comprising the steps of:
a. adding a sample containing analyte to a solution of the conjugate of the analyte containing fluorescent indicator;
b. adding an antibody specific to the analyte to the solution obtained in (a);
c. incubating the solution obtained in (b);
d. dispensing the incubate obtained in (c) over an immobilized anti-antibody;
e. reading the internal indicator fluorescent signal;
f. washing the unbound conjugate obtained in (d) with fluorogenic substrate;
g. reading the assay fluorescent signal; and
h. calculating the normal assay signal.

10. A method of normalizing the signal of an immunoassay involving the pipetting of liquid reagents and samples and comprising the steps of:
a. adding sample containing analyte to intrinsic factor supported on a solution of latex particles with fluorescent indicator;
b. incubating the solution obtained in (a);
c. dispersing the incubate obtained in (b) over a glass fiber matrix, such as GF/F;
d. reading the internal indicator fluorescent signal;
e. adding a conjugate to the dispersion obtained in (c);
f. washing the unbound conjugate obtained in (e) with a substrate;
g. reading the assay fluorescent signal; and
h. calculating the normalized assay signal.

11. The method of claims 9 or 10 wherein the immunoassay is a fluorescent immunoassay.

12. A method of determining estradiol in a sample comprising the steps of:
a. pipetting the estradiol sample into a solution of estradiol-alkaline phosphatase conjugate containing bis-(3-sulfopropyl)rhodamine piperazinamide acid;
b. pipetting the solution obtained in step (a) into rabbit antiestradiol antibody;
c. incubating the solution obtained in step (b);
d. dispersing the incubate obtained in step (c) over a glass fiber matrix, such as GF/F;
e. reading the indicator fluorescence signal of the dispersion obtained in step (d);
f. washing the unbound conjugate obtained in step (d) with 4-methylumbelliferyl phosphate fluorogenic substrate;
g. reading the assay fluorescent signal;
h. normalizing the estradiol assay signal by multiplying the ratio of the estradiol signal to the indicator signal by a constant.

13. A method of determining vitamin B₁₂ in a sample comprising the steps of:
a. pipetting vitamin B₁₂ sample into a solution of intrinsic factor supported on latex particles with bis-(3-sulfopropyl)rhodamine carboxylic acid;
b. incubating the solution obtained in (a);
c. dispensing the incubate obtained in (b) over a glass fiber matrix, such as GF/F;
d. reading the indicator fluorescence of the dispersion obtained in step (c);
e. pipetting vitamin B₁₂-alkaline phosphatase conjugate into the incubate obtained in step (c);
f. washing the unbound conjugate from the matrix of step (e) with 4-methylumbelliferyl phosphate fluorogenic substrate;
g. reading the generated fluorescence of step (f);
h. normalizing vitamin B₁₂ signal by mulitplying the ratio of the vitamin B₁₂ assay signal to the rhodamine signal by a constant.

14. The method of claim 12 or 13 wherein the method is performed on an analytical instrument.
